(19) Europäisches Patentamt
European Patent Office
Office européen des brevets

(11) Publication number: **0 356 366 A1**

(12) **EUROPEAN PATENT APPLICATION**

(21) Application number: 89500047.9

(22) Date of filing: 18.04.89

(51) Int. Cl.[5]: **C 07 D 277/48**
C 07 D 285/12,
C 07 C 323/64,
C 07 C 323/27,
C 07 C 311/49, C 07 C 311/00

(30) Priority: 18.07.88 ES 8802626

(43) Date of publication of application:
28.02.90 Bulletin 90/09

(84) Designated Contracting States:
AT CH DE ES FR GB IT LI NL SE

(71) Applicant: CENTRO MARGA PARA LA INVESTIGACION S.A.
Muntaner 212, 5o-510
E-08036 Barcelona (ES)

(72) Inventor: Ballester-Rodes, Montserrat
Marqués de Santa Ana 14, 2o
E-08023 Barcelona (ES)

Cabre-Castellvi, Francisco
Casp 114, esc.B.3o-1a
E-08010 Barcelona (ES)

Palomo-Nicolau, Francisco Eugenio
Dos de Mayo 34, 2o-1a
E-08190 Sant Cugat del vallés Barcelona (ES)

Cabre-Castellvi, Juan
Casp 114, esc.B.3o 1a
E-08010 Barcelona (ES)

Palomo-Coll, Antonio Luis
Escuelas Pias 18, 5o-1a
E-08017 Barcelona (ES)

(74) Representative: Ballester Rodés, Albert
Muntaner 212, 5o- 508
E-08036 Barcelona (ES)

(54) **A process for the preparation of N-sulfamyl-propionamidine derivatives.**

(57) N-sulfamyl-propionamidine derivatives are prepared by the reaction of 3-substituted-propionitriles with an oxonium hydrochloride, preferably of 1,4-dioxane, in the presence of sulfamide in a close system at pressures between 0.5 kg/cm and 4.0 kg/cm. These compounds are useful in the preparation of famotidine, an anti-ulcer agent.

EP 0 356 366 A1

## Description

### A process for the preparation of N-sulfamyl-propionamidine derivatives.

N-sulfamyl-propionamidine derivatives have been described for the preparation of the well known anti-ulcer agent famotidine.

In US patent 4.496.737, Spanish patent of introduction 545561, the process for preparing N-sulfamyl-3-(methylthio)propionamidine, comprises the step of converting the 3-methylthiopropionitrile to imidate and reaction with sulfamide, the overall yield being 20.7% of theory.

The use of N-sulfamyl-3-chloropropionamidine is disclosed in Spanish patent 526303 (Priority 7/10/1983), where there is no description of the preparation thereof nor antecedents of the synthesis thereof, nor are physico-chemical properties given allowing it to be identified. This lack of information is also found in Spanish patent 532239, where the use of N-sulfamyl-3(isothiourea)propionamidine is disclosed.

For the prior sulfamidines, Spanish patent 540352 teaches processes for preparation of 3-cloro and 3-isothiourea derivatives; the physico-chemical properties for indentification are not given and the examples are not sufficiently documented, which does not allow the results to be reproduced. Contrarywise to what is said in one of the examples, no precipitate is produced from the aqueous solution of N-sulfamyl-3-chloropropionamidine hydrochlo-ride, when adjusting the pH with sodium hydroxide.

Spanish patents 544597, 545913 and 549312 describe the reaction of 3-chloropropionimidate with sulfamide; no reference is made in any case to the physico-chemical properties for identification thereof.

The preparation of N-sulfamyl-3(isothiourea)propionamidine and of N-sulfamyl-3(mercapto)propionamidine is not described in Spanish patents 545510 and 549687 nor is any reference made to the physico-chemical properties. Both products are used for the preparation of famotidine. The lack of documentation makes it impossible to check the results given.

N-sulfamyl-3(2-guanidinothiazol-4-yl-methylthio)propionamidine, famotidine, was described for the first time in Spanish patents 487381 and 489222, where there is described the process comprising the sequence from nitrile to imidate and the reaction with sulfamide. US patent 4.496.737 also teaches the preparation of N-sulfamyl-acrylamidine starting from 3-methylthiopropionitrile.

European patent 0215639 A2 (Hungarian priority, 9/11/85) teaches a process for the preparation of N-sulfamyl-3-chloropropionamidine hydrochloride with its physico-chemical characteristics. The reaction of the above compound with 2-guanidinothiazol-4-yl-methylisothiourea is also described in Spanish patent application 8601790, for the preparation of famotidine, with a 52% overall yield.

The process for the preparation of N-sulfamyl-3-halopropionamidine, according to the description of the above European and Spanish patent applications, is effected by operating in an open system with a hydrogen chloride flow through the mixture of 3-chloropropionitrile and sulfamide; this method suffers from the following drawbacks:

1. A transfer and large loss of hydrogen chloride.
2. A complex industrial setup for the absorption of the free hydrogen chloride excess and preservation of the moisture causing the conversion of the nitrile into amide or acid, and for corrosion problems.
3. The process may not be carried out with solid propionitriles; it is limited to a liquid nitrile.
4. The yield obtained in 72% versus the sulfamide, but lower with respect to the hydrogen chloride.
5. The overall yield versus the famotidine obtained, is 52% of theory with respect to 100% N-sulfamyl-3-chloropropionamidine hydrochloride.

We have now discovered a process avoiding the previous difficulties, allowing application thereof to 3-substituted propionitriles related with famotidine.

The invention refers to N-sulfamyl-propionamidine derivatives of formula I,

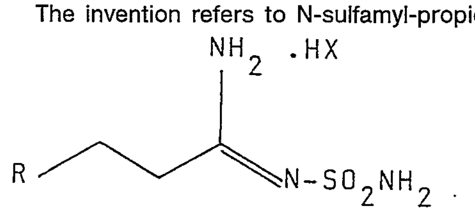

I

where R is a group selected from chlorine, bromine, mercapto, methylmercapto, isothiouronium (isothiourea salt), 5-methyl-1,3,4-thiadiazol-1-2-mercapto or 2-guanidinothiazol-4-yl-methylmercapto and X is selected from an atom of chlorine or bromine, which are easily prepared and of use as intermediates for conversion into famotidine, with the exception of the 2-guanidinothiazol derivative, which is famotidine of itself.

In accordance with the invention, the compounds of formula I may be prepared by reacting a propionitrile of formula II,

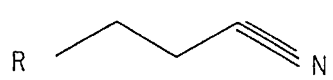

II

where R apart from the above meaning may be a hydroxyl or trialkylsilyloxy, with an oxonium salt, preferably

dioxane, of formula III,

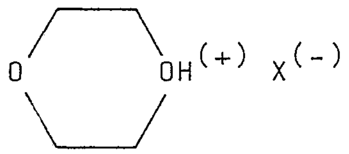

III

where X is as stated above, to form a compound of formula IV,

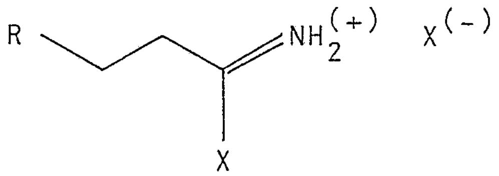

IV

where X is as stated above, which is reacted with a sulfamide of formula V,

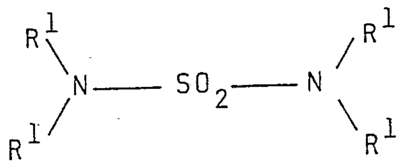

V

where $R^1$ is either atoms of hydrogen or $C_1$-$C_8$ trialkylsilyl groups, selected from trimethylsilyl, dimethylisopropylsilyl or dimethylterbutylsilyl, to form the desired formula I compound.

For the purposes of the invention, useful compounds representative of formula II, are 3-hydroxypropionitrile, 3-trimethylsilyloxypropionitrile, 3-chloropropionitrile, 3-bromopropionitrile, 3-mercaptopropionitrile, 3-(isothiourea)propionitrile, 3-(5-methyl-1,3,4-thiadiazol-2-mercapto)-propionitrile and 3-(2-guanidinothiazol-4-methylmercapto)propionitrile, all easily prepared from acrylonitrile and described in the scientific and technical literature.

The mercaptopropionitrile and salts of 3-(isothiourea)-propionitrile, have been prepared by the process of L. Bauer and T.L. Welsh (J.Org.Chem.,26, 1443-5, 1961) and 1,3,4-thiadiazole, according to Spanish patent 8702202.

In the case of 3-substituted propionitriles having amino groups, also useful are the derivative salts of sulfuric, trifluoromethanesulfonic, methanesulfonic, o-toluenesulfonic, p-toluenesulfonic, phenylsulfonic and other arenesulfonic acids, further to the hydrochloride. These acids may be used at a rate of one equivalent per amino group and another for the nitrile group, in order to facilitate a better dispersion of the phases of the mixture.

The 1,4-dioxane hydrochloride reagent of formula III, is suitably prepared by the absorption of at least one equivalent of hydrogen chloride in another of 1,4-dioxane, the mole ratio 1:1 of which is represented by the empirical formula $C_4H_9O_2X$, where X is an atom of chlorine. The composition contains 30% hydrogen chloride and 70% 1,4-dioxane.

The formula V compounds comprise sulfamide and N-silylated sulfamides such as N,N′-bis-trimethylsilylsulfamide. These, by reaction with oxonium hydrochloride, generate trimethylchlorosilane and sulfamide in the medium. In turn the trimethylchlorosilane eliminates the possible contaminating moisture, releasing hydrogen chloride. Water is harmful because it causes the formation of amide from compound IV.

The reaction between an oxonium hydrochloride of formula III with a nitrile of formula II, is effected in a closed system, preferably at a mole ratio of 4.0 to 15.0 per nitrile equivalent and at least one sulfamide equivalent. The preferred pressures lie between 0.5 kg/cm and 4.0 kg/cm at temperatures of 30° C to 75° C and particularly from 40°C to 60°C. The time may vary between 5 and 20 hours. For a longer time a lower temperature is required.

The isolation of N-sulfamyl-3-substituted-propionamidine is conducted by eliminating first the excess hydrogen chloride collected by dissolving the gas in 1,4-dioxane, and subsequent distillation of a part of 1,4-dioxane. Thereafter the mixture is cooled and in each particular case is diluted with dichloromethane and filtered, or diluted with water, with pH adjustment and filtration. The unreacted excess of nitrile and sulfamide may be recovered for subsequent use.

The alternative ways of reaction of the 2-guanidino-4-chloromethyl-thiazole with 3-isothiourea-propionitrile, 3-chloropropionimidate and N-sulfamyl-3-(isothiourea)-propionamidine, in the presence of sodium hydroxide and under the experimental conditions described in Spanish patent 532239 (Inke S.A., Example 2), Spanish patent 545510 (Example 1) and Spanish patent 544597 (sole example), do not give the desired product, famotidine. In all cases thiourea and other compounds are released.

For the purpose of the invention, the preferred nitriles are those yielding N-sulfamyl-3-chloropropionamidine and those which gives famotidine itself. The process used offers the following advantages:

1. The reactants of the preparation process are available and used in industry.

2. The product is readily isolated by filtration, with 86% yield and high purity.

3. The excess hydrogen chloride, nitrile and optionally the 1,4-dioxane are recovered for recycling.

4. The conversion of 2-guanidino-4-mercaptomethyl-thiazole in famotidine, using the salt 1,8-diazabicyclo[5.4.0]undec-7-ene (DBU), described in Spanish patent 8800205, is achieved in a short time at room temperature with a yield of 88 to 92% of theory.

5. Starting out from 3-(2-guanidinothiazol-4-yl-methylthio)propionitrile, a solid product prepared according to Spanish patent 8700719, famotidine is obtained in a single stage.

In accordance with a further aspect of the invention, there is provided a process for the preparation of solutions of a compound of formula VI.

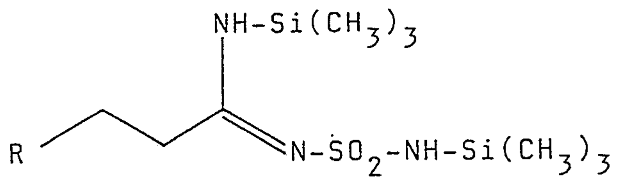

VI

where R is preferably chlorine. To this end dimethylformamide, dimethylacetamide, acetonitrile or mixtures thereof with cosolvents such as dichloromethane or chloroform are used.

The silylating agent is selected from the group formed by 3-trimethylsilyl-2-oxazolidinone, trimethylsilyl-acetamide, hexamethyldisilazane, among other known reagents described in the technical literature. The reaction of sulfamidine VI, where R = chlorine, with the 2-guanidino-4-mercaptomethyl-thiazole DBU salt gives a solution of N, N′-bis-trimethylsilylated famotidine, from which the anti-ulcer agent is isolated by lysis in methanol and precipitation with dichloromethane, giving the high melting point polymorph (Spanish patents 8800205 and 872020).

Example 1

N-sulfamyl-3-(5-methyl-1,3,4-thiadiazol-2-thio)propionamide

3-(5-methyl-1,3,4-thiadiazol-2-thio)propionitrile (3.64 g; 2.0 cmoles) and sulfamide (1.92 g; 2.0 cmoles) were added to 1,4-dioxane hydrochloride (30.0 g 30% w/w HCl). The mixture was heated in a closed system with stirring to 45°-50°C under a pressure of 0.5-1.0 kp/cm. After 3 hours reaction, a slightly yellow solution was produced. After further 2 hours, the hydrogen chloride was gradually removed and picked up on 1,4-dioxane. The resulting mass was gradually added over aqueous ammonia (10%; 100 ml) with cooling to 0°-5° C. After 30 minutes at the same temperature, the mass was filtered, washed with water and dried to give the title compound (4.30 g) with 76% yield, m.p. - 162°-165°C. I.R. spectrum identical to that of a pure sample.

Example 2

N-sulfamyl-3-(2-guanidinothiazol-4-yl-methylthio)propionamidine

(2-guanidinothiazol-4-yl-methylthio)-isothiourea (6.06 g; 2.0 cmoles) dihydrochloride was dissolved in methanol (10.0 ml; containing 0.36 g of water; 2.0 cmoles). Thereafter a solution of sodium methoxide in methanol (12.80 ml, 27% w/v; 6.4 cmoles) was added, the temperature being held at 25°C. A sodium chloride suspension resulted. After 15 min. at 25°C, a catalytic amount (0.2 cmole) of an amidine such as 3-(2-guanidinothiazol-4-yl-methylthio)-propionamidine dihydrochloride or tetramethylguanidine, followed by N,N-dimethylformamide (5.0 ml) and N-sulfamyl-3-(5-methyl-1,3,4,-thiadiazol-2-thio)-propionamidine (6.22 g; 2.2 cmoles) prepared according to Example 1 was added. The suspension was heated to reflux (62°C) for a period of time (30 min.; negative test with nitroprussiate) and was thereafter cooled to room temperature. Thereafter it was poured over a mixture of water (70 ml) and dichloromethane (40 ml) to give an oil which after being decanted and suspended in methanol, gave the title compound (0.9-1.0 g), yield 13.0%, with the I.R. spectrum corresponding to that of the low melting point polymorph (160°-162°C).

The dichloromethane was evaporated from the residual liquors and 27±3% of famotidine was quantified by thin layer chromatography. This represents an overall yield of 40% of theory.

Example 3

N-sulfamyl-3-chloro-propionamidine hydrochloride

Sulfamide (13.46 g; 14.0 cmoles) was added to a solution of 3-chloropropionitrile (41.8 ml; 53.2 cmoles) in 1,4-dioxane hydrochloride (114.0 g, 30% w/w). The mixture was heated under stirring to a temperature of 55°-60°C for four hours at a pressure of 1.0-1.5 kp/cm, which was held by the addition of more hydrogen chloride. A slightly cloudy solution resulted. The excess acid was removed, and collected over 1,4-dioxane (100 ml), cold, the mixture being exhausted by heating to 80°-85°C. The removal of the hydrogen chloride remains was completed by evaporation at reduced pressure. The resulting solution was cooled in a water-ice bath (0°-5°C) and diluted with dichloromethane (100 ml) to give an oil, which was solidified by stirring at room temperature over a period of time (30 min.). It was cooled to 0°-5°C again for 45 min., filtered, washed with dioxane (twice, 30 ml) and with dichloromethane (twice, 40 ml). After drying, it gave the title compound (26,8

g), yield 86.2% of theory, m.p.: (136° C, with softening) 139°-142° C and I.R. (KBr), νcm$^{-1}$: 3340, 3300, 3000, 2480, 1680, 1630, 1505, 1170, 920 and 815 (most representative readings). Hydrogen chloride recovered, 20.0 g.

Example 4

N-sulfamyl-3-(2-guanidinothiazol-4-yl-methylthio)-propiononamidine

A solution of sodium hydroxide (6.0 g; 15.0 cmoles) in methanol-water (1:1, 100 ml) was added to a solution of N-sulfamyl-3-chloropropionamidine hydrochloride (33.3 g; 15.0 cmoles) in water (100 ml), with cooling to 0°-5° C. Thereafter the 1,8-diazabicyclo[5.4.0]undec-7-ene salt of 2-guanidine-4-mercaptomethyl-thiazol (51.0 g; 15.0 cmoles) was added and the temperature was allowed to rise to 10° C. The mixture was stirred for a time (2 hours) under these conditions and then at 25°-30° C for about 30 minutes. 60 minutes after initiation of the reaction, precipitation started.

At the end of precipitation (negative nitroprussiate test), the mixture was held at 0°-5° C (2 hours). It was filtered, washed with water-methanol (3:1, 150 ml) and dried, to give the title compound (45.6 g), yield 90.0% of theory, m.p.: 161°-164° C. I.R. spectrum identical to that of a pure sample.

Example 5

A. 3-(2-guanidinothiazol-4-yl-methylthio)-propionitrile trifluoromethanesulfonate

Trifluoromethanesulfonic acid (3.87 ml; 4.4 cmoles) was added to a suspension of 3-(2-guanidinothiazol-4-yl-methylthio)-propionitrile (9.64 g; 4.0 cmoles) in 1,4-dioxane (40 ml) at room temperature with vigorous stirring. The temperature rose to 32° C, giving a solution from which the product crystallized by cooling to 0°-5° C, more quickly by triphlate seeding. After 30 min. at the same temperature, the product was filtered, washed in anhydrous dioxane (20 ml) and dichloromethane (20 ml). The result was the title compound (14.53 g), yield 92.7%, K.F. = 0.12% and m.p. = 123.5°-125.5° C. I.R. (KBr) νcm$^{-1}$: 3400, 3160, 3000, 2240, 1680, 1610, 1505 (doublet) 1300 (wide with subbands), 1040; these were the most representative readings. Correct microanalysis.

B. 3-(2-guanidinothiazol-4-yl-methylthio)-propionitrile methanesulfonate

Methanesulfonic acid (0.70 ml, 4% $H_2O$ (K.F.), 1.01 cmole) was added in one shot to a suspension of 3-(2-guanidino thiazol-4-yl-methylthio)-propionitrile (2.41 g, 1.0 cmole) in dioxane (10.0 ml). The initial temperature of 25° C rose to 38° C. There was a partial solution followed by precipitation of a dense white solid. After stirring the mixture (15 min. at 20°-25° C) it was filtered, washed with dioxane (5 ml) and dichloromethane (5 ml), and after drying it gave the title compound (3.33 g), yield 98.8%, K.F. 0.17% and m.p. = 144°-147° C. I.R. (KBr) νcm$^{-1}$: 3200 (wide with two subbands), 2910, 2230, 1700, 1615 and 1040; these were the most representative readings. The rapid m.p. determination gave a range of 145°-150° C. Correct microanalysis.

C. 3-(2-guanidinothiazol-4-yl-methylthio)-propionitrile p-toluenesulfonate

p-Toluenesulfonic monohydrate (1.90 g; 1.0 cmole) was dissolved in dioxane (20 ml) at room temperature and the guanidinothiazole (2.41 g; 1.0 cmole) was added in one shot. The result was a complete solution, with exothermic reaction (temperature rose from 23° to 30° C). The mixture was stirred at room temperature and a slow precipitation started. After a period of time (30 min.), a very thick slurry was formed. It was diluted with ethyl ether (20 ml) and stirred for 60 min. at the same temperature. It was filtered, washed with ethyl ether (20 ml) and dried to give the title compound (3.62 g). Yield 87.4% of theory, K.F. 0.22% and m.p. = 139°-142° C. Characteristic readings I.R. (KBr) νcm$^{-1}$: 2020 (-C=N), 1000 and 1020 ($-SO_3H$).

Following the above process, other arenesulfonates such as benzenesulfonate, o-methylbenzenesulfonate and salts of the sulfate itself were prepared.

Example 6

N-sulfamyl-3-(2-guanidinothiazol-4-yl-methylthio)- propionamidine

3-(2-Guanidinothiazol-4-yl-methylthio)-propionitrile (7.23; 3.0 cmoles) was added with stirring and cooling in an ice-water bath to 1,4-dioxane hydrochloride (45.0 g, 30% w/w HCl), followed by sufamide (3.45 g, 3.6 cmoles). The mixture was heated for 60 min. at a temperature of 65°-70° C at a pressure of 2.0 kp/cm. Thereafter it was cooled to 40°-50° C and the liquors were decanted. The residue was dissolved in dimethylformamide (6 ml) with stirring at 45°-50° C. The resulting viscous solution was poured over a solution of ammonia (45.0 ml, 8%), the temperature being held at 0°-5° C.

To recover the unconverted product, the suspension formed was treated with a sodium methoxide solution (30.0 ml, 27.3%, 1.5 cmoles) and after stirring for a time (15 min. 0°-5° C) it was filtered, washed with water (30 ml) and dried to give guanidinethiazole as starting product (3.51 g; 1.32 cmoles), yield 44%, correct I.R.

Acetic acid was added to the above filtration liquors to pH = 7.5 (approximately 0.9 ml; 1.5 cmoles). The mixture was stirred for a time (2 hours at 5°-10° C), and was filtered, washed with water (30 ml) and dried to give the title compound 4.0 g; yield = 40%). The overall yield relative to the starting product recovered is 60.7% m.p. = 157°-161° C.

Example 7

N-sulfamyl-3-(2-guanidinothiazol-4-yl-methylthio)-propionamidine

3-(2-Guanidinothiazol-4-yl-methylthio)-propionitrile trifluoromethanesulfonate (11.73 g, 3.0 cmoles) and sulfamide (5.76 g, 6.0 cmoles) was added to 1,4-dioxane hydrochloride (45.0 g, 33%; 14.0 cmoles). The mixture was stirred (insoluble slurry) and heated to 35°-40°C under a pressure of 1.5-2.0 kp/cm, for a time (20 hours).

Thereafter the hydrogen chloride was removed by heating at reduced pressure and collected over dioxane. The resulting mixture was added at 35°-40°C over an ammonia solution (30 ml, 13.0%) chilled to 0°C; it was stirred at this temperature for 3 hours. The precipitate was filtered, washed with water (30 ml) and dried, to give the title product (7.31 g), yield 70.2% of theory, m.p. = 157°-161°C.

Example 8

3-(2-guanidinothiazol-4-yl-methylthio-propionitrile

1,8-diazabicyclo[5.4.0]undec-7-ene (DBU, 29.9 ml; 20 cmoles) was added to a suspension of 3-(isothiourea)-propionitrile hydrochloride (16.6 g; 10.0 cmoles) in acetonitrile (100 ml) with water (2.7 ml; 15.0 cmoles), chilled to 0°-5°C, under stirring. The mixture was stirred for a time (30 min.) between 0°-10°C. Thereafter there was added first 2-guanidino-4-hydroxy-4-chloromethyl-thiazole hydrochloride (24.5 g, 10.0 cmoles) and then DBU (14.9 ml, 10 cmoles), causing the temperature to rise to 20°C. The mixture was held at this temperature under stirring for a time (30 min.). The solvent was evaporated off at reduced pressure and the residue was diluted with water (400 ml), with stirring at 0°-10°C. The precipitate was filtered, washed with water (2x100 ml) and dried to give the title compound (19.8 g) with a crystalline appearance and yield 82% of theory, K. F. 0.8%; m.p. = 126°-128°C. I.R. spectrum correct.

Following the method described in Spanish patent 532239 (Example 2), and replacing the sulfamidine 3-isothiourea salt with the corresponding propionitrile 3-isothiourea salt (much more active), the title compound could not be obtained.

Example 9

N-sulfamyl-3-(isothiourea)-propionamidine hydrochloride triflate

Following Example 1 and replacing the 3-(5-methyl-1,3,4-thiadiazol-2-thio)-propionitrile with 3-(isothiourea)-propionitrile trifluoromethanesulfonate (triflate) (5.60 g, 2.0 cmoles, oil) under the same conditions, after removal of the hydrogen chloride solution of the title compound was obtained. The same result was obtained when using the methanesulfonate.

3-(Isothiourea)-propionitrile hydrochloride and p-toluenesulfonate were prepared according to the process of L. Bauer and T.L. Welsh (J. Org. Chem. 26, 1443-5; 1961).

The starting triflate was prepared as follows:

Trifluoromethanesulfonic acid (5.13 g, 3.42 cmoles) was added gradually to a suspension of 2.60 g (3.42 cmoles) of thiourea in absolute ethanol (30 ml), causing the temperature to rise. The temperature was raised to 70°C and acrylonitrile (1.82 g, 3.42 cmoles) was added. After a period of time (2 hours) at 70°C, the solvent was removed by evaporation at reduced pressure to give the title compound (9.54 g, oil) with quantitative yield. I.R. spectrum correct.

Following the same process and using anhydrous methanesulfonic acid (3.28 g, 3.42 cmoles) the product obtained was 3-(isothiourea)-propionitrile methanesulfonate, yield 80%, m.p. = 144°-148°C (washed with ethanol), and correct I.R. spectrum.

Example 10

N-sulfamyl-3-mercapto-propionamidine hydrochloride

Following Example 1, 3-mercaptopropionitrile. prepared according to the method of L. Bauer and T. L. Welsh (J. Org. Chem. 26, 1443-5, 1961) was used. After the excess hydrogen chloride had been removed and collected, the resulting product was a mixture of the title compound in dioxane. I.R. spectrum correct, positive nitroprussiate test.

Example 11

N-sulfamyl-3-chloropropionamidine hydrochloride

Following Example 3, and replacing the sulfamide with N, N-bis-trimethysilylsulfamide (33.6 g, 14.0 cmoles), the amount of 1,4-dioxane hydrochloride per 150.0 g at 30% was used. The pressure was held at 2.5 kp/cm. Thereafter the trimethylchlorosilane and hydrogen chloride were removed by destillation to give a solution which when treated as described in Example 3 gave the title compound on isolation, with similar characteristics and yield.

Example 12

N-sulfamyl-3-(2-guanidinothiazol-4-yl-methylthio)-propionamidine

The 1.8-diazabicyclo[5.4.0]undec-7-ene salt of 2-guanidino-4-mercaptomethyl-thiazole (5.10 g, 1.50 cmoles) was added to a solution of N,N′-bis-trimethylsilyl-N-sulfamyl-propionamidine (containing 1.50 cmoles) in dimethylformamide (20 ml). The mixture was stirred at 35° C-40° C until a negative nitroprussiate result was obtained. Thereafter methanol (1.0 ml methanol, 3.0 cmoles) was added. The solution was diluted with chloro form (75 ml) and stirred in a water-ice bath. The liquid was decanted and more chloroform was added to break down the solid. The precipitate was isolated, washed with dichloromethane, water and methanol. On drying, it gave the title compound (4.0 g), yield 80% and m.p. = 167°-169° C.

A solution of bisilylated N-sulfamyl-propionamidine was prepared by heating 2.0 equivalents of 3-trimethylsilyl-2-oxazolidinone and removing the trimethylchlorosilane formed together with the bisilylated compound by distillation at reduced pressure.

Example 13

N-sulfamyl-3-(isothiourea)-propionamidine dihydrochloride

3-(Isothiourea) propionitrile hydrochloride (4.95 g; 3.0 cmoles) was suspended in 1,4-dioxane hydrochloride (450 g; 33% w/w) and the mixture was stirred and heated to 45°-50° C at a pressure of 2.0 kp/cm, for 20 hours. Thereafter, the excess hydrogen chloride was removed, being collected over 1,4-dioxane. The precipitate was filtered and suspended in 1,4-dioxane (50 ml), and stirred for a period of time (30 min.). The mixture was filtered and washed successively with dioxane (30 ml) and dichloromethane (30 ml). After drying, the title compound (4.56 g; 85% purity as dihydrochloride) was obtained, with m.p. = 115°-141° C (without recrystallizing). I.R. (KBr) $\nu cm^{-1}$: 3600-2400 (with two subbands at 3260 and 3070), 1650 (C=N, wide), 1500 (weak), 1440, 1400, 1180 with subband at 1140 ($-SO_2-$), 920 and 800.

## Claims

1. A process for the preparation of N-sulfamyl-propionamidine derivatives of the following formula:

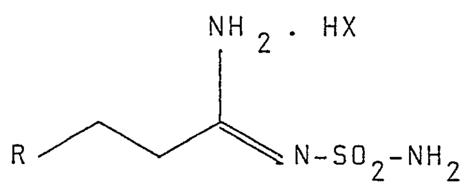

I

where R is a halogen such as chlorine or bromine, a group selected from mercapto, methylmercapto, isothiouronium, 5-methyl-1,3,4-thiadiazol-2-mercapto or 2-guanidinothiazol-4-methylmercapto, and X is an atom of chlorine or bromine, characterized in that 3-subsituted-propionitrile having the formula II,

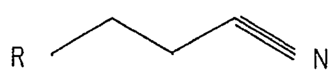

II

where R is as stated above, is reacted with an oxonium salt of formula III,

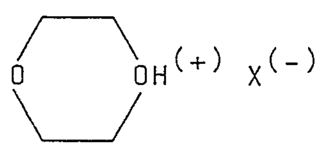

III

where X is an atom of chlorine or bromine, to form a salt of the imidoyl halide of formula IV,

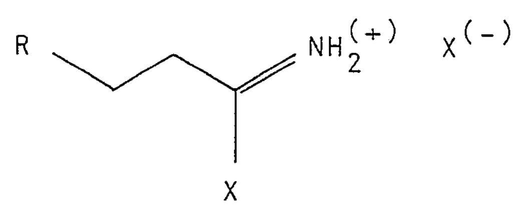

IV

where R and X are as stated above, which is reacted with a sulfamide of formula V,

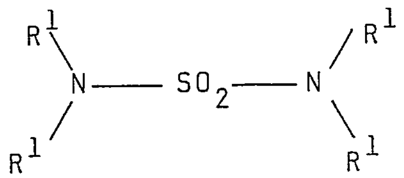

V

where $R^1$ is an atom of hydrogen or a $C_1$ -$C_8$ trialkylsilyl group, preferably trimethylsilyl, triethylsilyl, dimethyltertbutylsilyl or dimethylisopropylsilyl, to give a compound of formula I.

2. The process of claim 1, characterized in that 1.0 to 3.0 moles of 3-substituted-propionitrile are reacted with 4.0 to 15.0 moles of 1,4-dioxane hydrochloride in the presence of 1.0 mole to 3.0 moles of sulfamide or N, N′ -bis-trimethylsilyl sulfamide, in a closed system at a pressure of 0.5 to 4.0 kg/cm, at a temperature of 35° C to 75° C and preferably from 40° C to 70° C, for a period of time of 4 to 20 hours.

3. The process of claim 1, for the preparation of solutions of N-sulfamyl-propionamidine of formula VI,

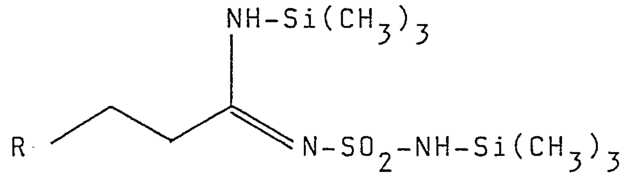

VI

where R is preferably a chlorine atom, characterized in that a compound of formula I, in dimethylformamide, dimethylacetamide, acetonitrile or mixtures thereof with a cosolvent selected from dichloromethane or chloroform, is reacted with a silylating agent, preferably 3-trimethylsilyl-2-oxazolidinone, hexamethyldisilazane or trimethylsilylacetamide, at a temperature of 60° C to 75° C.

4. The process of claim 1, characterized in that the excess hydrogen chloride, nitrile, sulfamide and 1,4-dioxane are recycled in the process.

5. The process of claim 1, characterized in that the compound of formula II, 3-(2-guanidinothiazol-4-yl-methylthio)-propionitrile, is selected as sulfuric or sulfonic acid salt, preferably from the sulfate or from the group formed by methanesulfonic, toluenesulfonic, trifluoromethanesulfonic, benzenosulfonic or phenolsulfonic acids.

6. The process of claim 1, for the preparation of famotidine, characterized in that one equivalent of 3-(2-guanidinothiazol-4-yl-methylthio)propionitrile trifluoromethanesulfonate is reacted with up to 15.0 equivalents of 1,4-dioxane hydrochloride at a pressure of 0.5 to 4.0 kg/cm, with up to three equivalents of sulfamide at 35° -40° C for 20 hours.

7. A process for the preparation of famotidine, comprising the S-alkylation of the 1,8-diazabicyclo[5.4.0]un-dec-7-ene salt of 2-guanidino-4-mercaptomethyl-thiazole with an N-sulfamylpropionamidine of formula I or formula VI as defined in claim 1, where R and X are an atom of chlorine, at room temperature, in the presence of one equivalent of an organic base.

European Patent Office

# EUROPEAN SEARCH REPORT

Application number: EP 89500047.9

**DOCUMENTS CONSIDERED TO BE RELEVANT**

| Category | Citation of document with indication, where appropriate, of relevant passages | Relevant to claim | CLASSIFICATION OF THE APPLICATION (Int. Cl.4) |
|---|---|---|---|
| P,A | EP - A1 - 0 284 536 (CENTRO MARGA PARA LA INVESTIGACION S.A.) * Claim 1 * | 1,6,7 | C 07 D 277/48<br>C 07 D 285/12<br>C 07 C 323/64<br>C 07 C 323/27<br>C 07 C 311/49<br>C 07 C 311/00 |
| D,A | DE - A1 - 2 951 675 (YAMANOUCHI PHARMACEUTICAL CO.) * Claim 8 * | 1,6,7 | |
| A | DE - A1 - 3 644 246 (J.URIACH & CIA. S.A.) * Claim 1 * | 1,6,7 | |
| D,A | EP - A2/A3 - 0 215 639 (RICHTER GEDEON VEGYESZETI GYAR. R.T.) * Claims 1,3,8 * | 1 | |

TECHNICAL FIELDS SEARCHED (Int. Cl.4)

C 07 D 277/00
C 07 D 285/00
C 07 C 149/00
C 07 C 143/00

The present search report has been drawn up for all claims

| Place of search | Date of completion of the search | Examiner |
|---|---|---|
| VIENNA | 21-07-1989 | BRUS |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document